# EUROPÄISCHE PATENTSCHRIFT

(11) **EP 0 820 984 B1**
(45) Veröffentlichungstag und Bekanntmachung des Hinweises auf die Patenterteilung: **27.03.2002**
(21) Anmeldenummer: 97112747.7
(22) Anmeldetag: 24.07.1997
(51) Int. Cl.: C07D 207/44, G01N 33/543

(54) **Neue Aktivierte Amphetamine**
Activated amphetamines
Amphétamines activées

(30) Priorität: 25.07.1996 DE 19630102
(43) Veröffentlichungstag der Anmeldung: 28.01.1998
(73) Patentinhaber: Roche Diagnostics GmbH, 68305 Mannheim (DE)
(72) Erfinder: Huber, Erasmus, Dr., 86923 Finning (DE); Klein, Christian, Dr., 82362 Weilheim (DE); Vogel, Rudolf, Dr., 82362 Weilheim (DE); Zink, Bruno, 82449 Uffing (DE); Rollinger, Wolfgang, Dr., 82398 Polling (DE)
(74) Vertreter: Weiss, Wolfgang, Dipl.-Chem. Dr.

(56) Entgegenhaltungen:
- EP-A- 0 386 644
- WO-A-90/15798
- US-A- 4 329 281
- Pierce ImmunoTechnology Catalog & Handbook, Kopien zu den Reagentien DSS, BS3, DSP und DTSSP.
- Molecular BioSciences Catalog 1996, Kopie zu den Reagenten SMCC bis SATP.

## Beschreibung

Die Erfindung betrifft aktivierte Amphetaminderivate, ein Verfahren zu deren Herstellung sowie deren Verwendung zur Herstellung von Amphetamingruppen-tragenden Immunogenen oder Nachweiskonjugaten. Ein weiterer Gegenstand der Erfindung sind neue Amphetamingruppen-tragende Konjugate, ein Verfahren zu deren Herstellung sowie deren Verwendung zur Erzeugung von Antikörpern oder zur Bestimmung von Amphetamin bzw. von Derivaten davon.

Amphetamin und verwandte Substanzen davon (nachfolgend der Einfachheit halber zumeist unter dem Begriff "Amphetamine" zusammengefaßt) gehören zu der Gruppe der Psychoanaleptika und weisen sympathomimetische Wirkung und suchtfördernde Eigenschaften auf. Sie besitzen daher ein großes Mißbrauchspotential und in diesem Zusammenhang besteht ein Bedarf an analytischen Bestimmungs- und Nachweismethoden.

In den vergangenen Jahren wurden zur Kontrolle des Drogenmißbrauchs insbesondere immunologische Verfahren entwickelt, da diese eine schnelle und kostengünstige Methode zum Nachweis von Betäubungsmitteln in Körperflüssigkeiten darstellen. Zur Erzeugung von Antikörpern, die in derartigen Immunassays geeignet verwendet werden können, ist es erforderlich, ein Immunogen durch Kopplung von Amphetamin an einen geeigneten Träger herzustellen, danach in an sich bekannter Weise Antikörper zu erzeugen und diese Antikörper zu isolieren.

Konjugate von Amphetaminen und Trägersubstanzen sind aus dem Stand der Technik bekannt. In einer Reihe von Veröffentlichungen (1-9) werden Konjugate von Amphetaminen mit Polypeptiden bzw. Nachweisgruppen wie Fluoreszenzfarbstoffen beschrieben, wobei die Kopplung über einen Linker erfolgt, der an die Ethylamin-Seitengruppe des Amphetaminmoleküls gebunden ist. Antikörper, die nach Immunisierung mit einem derartigen Immunogen erhalten werden, zeigen jedoch eine hohe Kreuzreaktivität zu verschiedenen Amphetaminderivaten, die sich in der Substitution der Ethylaminseitenkette unterscheiden (22,23). Sie sind daher für die Analytik nur begrenzt geeignet, insbesondere wenn beispielsweise zwischen Amphetamin, Methamphetamin und Ephedrin zu differenzieren ist.

Weiterhin ist aus dem Stand der Technik die Herstellung von Amphetamin-tragenden Konjugaten bekannt, wobei die Kopplung über eine Substitution am aromatischen Ring erfolgt (10-21). Bei Einführung eines Substituenten, der eine aktivierbare Gruppe besitzt, am aromatischen Ringsystem muß jedoch bei Aktivierung vor dem Konjugationsschritt die Aminofunktion der Amphetaminseitenkette (primäre Aminogruppe bei Amphetamin, sekundäre bei Methamphetamin) durch Einführung einer Schutzgruppe blockiert werden (11,21). Nach erfolgter Konjugation muß diese Schutzgruppe zur Wiederherstellung des gewünschten Amphetamin-Epitops entfernt werden. Insbesondere bei Verwendung eines makromolekularen Trägers wie etwa bei einem Polypeptid ist die vollständige Abspaltung der Schutzgruppe nicht nachweisbar. Weiterhin kann die Funktion des Konjugats aufgrund der zur Schutzgruppenabspaltung erforderlichen Reaktionsbedingungen (in der Regel starke pH-Änderungen durch Säure oder Base oder Einwirkung von stark oxidierenden oder reduzierenden Agenzien) beinträchtigt werden (z.B. Denaturierung eines Reporterenzyms).

Aoki et al. (13) beschreiben einen Immunoassay für Methamphetamin unter Verwendung eines neuen Antikörpers. Der Antikörper wurde erhalten nach Immunisierung mit einem Konjugat, welches hergestellt wurde durch ein Einschritt-Verfahren, wobei Mercaptosuccinyl-BSA, p- oder o-Aminomethamphetamin und 4-(Maleimidomethyl)cyclohexan-1-carbonsäuresuccinimidester (MCCS) umgesetzt wurden. Bei dieser Umsetzung kann die Succinimidgruppe mit der Aminogruppe am aromatischen Ring oder/und mit der Aminogruppe in der Ethylamin-Seitenkette reagieren, so daß kein spezifisches Produkt erhalten wid. Die Herstellung von stabilen Amphetaminderivaten, die eine aktivierte Linkerfunktion am aromatischen Ringsystem tragen, ist ebenfalls nicht offenbart.

WO90/15798 offenbart heterobifunktionelle Reagenzien, die zur Herstellung von Hapten-Peptid-Derivaten geeignet sind. Diese Reagenzien sind Aminoalkylmaleimide, wobei die Verknüpfung mit dem Hapten durch Amidierung von Carboxylgruppen erfolgt. Als geeignete Haptene werden beispielsweise Phenobarbital, Diphenylhydantoin, Carbamazepin, Valproinsäure, Thyroxin (T4), Trijodthyronin (T3), Östron, Östradiol, Progesteron, Testosteron, Aldosteron, Folsäure, Methyltetrahydrofolsäure oder Cyancobalamin (Vitamin B₁₂) genannt; Amphetamine werden jedoch nicht erwähnt.

Aufgabe der vorliegenden Erfindung ist es, aktivierte aber stabil lagerfähige Amphetaminderivate bereitzustellen, die es ermöglichen diese Haptene über das aromatische Ringsystem an einen Konjugationspartner zu koppeln, ohne daß nach Konjugation eine Aktivierung wie etwa eine Abspaltung der Schutzgruppe außerhalb eines pH-Bereichs von 6,0 bis 8,5 erforderlich wird, welche zum Verlust der biologischen Aktivität führen kann.

Ein weiterer Gegenstand der Erfindung sind Konjugate, die unter Verwendung der erfindungsgemäßen Amphetaminderivate hergestellt sind. Ein nochmals weiterer Gegenstand ist ein Verfahren zur Herstellung des erfindungsgemäßen Amphetaminderivats bzw. des Amphetamin-tragenden Kohjugats.

Ein nochmals weiterer Gegenstand ist die Verwendung eines erfindungsgemäßen Konjugats in einem Verfahren zur Gewinnung von gegen Amphetamine gerichteten Antikörpern bzw. zur Bestimmung von Amphetaminen.

Gelöst wird die erfindungsgemäße Aufgabe durch ein aktiviertes Amphetaminderivat, gekennzeichnet durch die Strukturformel 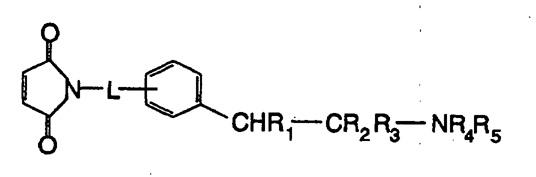 wobei bei R₁ H oder OH ist,
R₂, R₃, R₄ und R₅ unabhängig voneinander H, CH₃ oder C₂H₅ sind, und L ein Linker mit einer Kettenlänge von 4-30 Atomen ist, oder ein Salz davon.

Die Linkerkette enthält gegebenenfalls substituierte C-Atome und kann Heteroatome wie N, O und S enthalten, die unabhängig voneinander substituiert sein können. Beispiele für substituierte Atome in der Linkerkette sind -C(R₆)₂-, -NR₆-, -CR₆OR₆-und -C(O)-, wobei R₆ jeweils unabhängig voneinander bevorzugt Wasserstoff oder C₁ bis C₅ Alkyl bedeutet. Der Linker kann auch Mehrfachbindungen enthalten, d.h. zwei benachbarte Atome im Linker können -CR₆=CR₆-, -C≡C- oder -CR₆=N- sein.

Bevorzugt weist L eine Kettenlänge von 6 bis 20, stärker bevorzugt eine Länge von 8 bis 18 Atomen auf.

Im allgemeinen werden Linker verwendet werden, die im wesentlichen unsubstituiert sind und in einer Ausführungsform sind die Atome in der Linkerkette unabhängig voneinander bei jedem Vorkommen ausgewählt aus -CH₂-, -CH=, -C≡, -NH-, =N-, -CHOH-, -C(O)-, -O- und -S-. In einer anderen Ausführungsform sind innerhalb der linearen Linkerkette keine Mehrfachbindungen vorhanden.

Ein wesentlicher Vorteil der erfindungsgemäßen aktivierten Amphetaminderivate besteht darin, daß bei späterer Umsetzung mit einem geeigneten Konjugationspartner ein Schutz der Amingruppe in der Ethylamin-Seitenkette nicht erforderlich ist.

Dadurch kann einerseits gewährleistet werden, daß die Amphetamingruppe vollständig aktiv vorliegt und andererseits werden weitere Arbeitsschritte (Schutzgruppenabspaltung) vermieden, die eine Beeinträchtigung der Aktivität des Konjugationspartners zur Folge haben können. Die erfindungsgemäßen Amphetaminderivate sind daher besonders vorteilhaft zu verwenden, wenn mindestens einer von R₄ und R₅ H ist.

Bevorzugt ist der Linker in p-Position zu der Ethylamin-Seitenkette angeordnet. Eine Anordnung in o- oder m-Position ist jedoch ebenfalls möglich. Beste Ergebnisse werden für diejenigen Amphetamine erzielt, bei denen durch die räumliche Verknüpfung des Linkers mit dem speziellen Amphetamin eine möglichst geringe Beeinflussung der Zugänglichkeit des Amphetaminepitops erreicht wird.

In einer bevorzugten Ausführungsform ist L
[-(CH₂)ₒ-NH-C(O)-]ₚ-(CH₂)_{q}-X-, wobei o unabhängig bei jedem Vorkommen 2 bis 6 ist, p 1 oder 2 ist, q 2 bis 10 ist und X ausgewählt ist aus O, NH und einer Bindung. Bevorzugt ist X eine Bindung.

In der vorliegenden Erfindung haben sich insbesondere aktivierte Amphetaminderivate, bei denen L die Struktur -(CH₂)ₒ-NH-C(O)-(CH₂)_{q}- aufweist, wobei o und q die oben angegebenen Bedeutungen haben, als geeignet herausgestellt. In einer Ausführungsform ist o 2 bis 4, bevorzugt 2 und q ist 3 bis 8, bevorzugt 4 bis 6.

Die erfindungsgemäß aktivierten Amphetaminderivate sind zur Verwendung mit den unterschiedlichen Amphetaminen entsprechend der Definition der Reste R₁ bis R₅ geeignet. In der Praxis besonders wichtige Amphetaminderivate sind diejenigen, bei denen R₃ und R₅ jeweils H sind, also die aktivierten Derivate von beispielsweise Amphetamin, Methamphetamin, Ephedrin, β-Phenylethylamin und Norephedrin. Besonders von Bedeutung sind die aktivierten Derivate von Amphetamin und Methamphetamin.

Ein weiterer Gegenstand der Erfindung ist ein Konjugat, umfassend mindestens eine Amphetamingruppe und einen Konjugationspartner, welches dadurch gekennzeichnet ist, daß es durch Umsetzen der Maleinimidgruppe eines erfindungsgemäßen aktivierten Amphetaminderivats mit einer SH-Gruppe des Konjugationspartner erhältlich ist. Vorzugsweise sind sowohl R₄ als auch R₅ H.

Die SH-Gruppe des Konjugationspartners kann eine native (natürlich vorkommende) SH-Gruppe sein oder/und durch Derivatisierüng des Konjugationspartners erzeugt worden sein.

Ein weiterer Gegenstand der Erfindung ist ein Konjugat mit der Strukturformel 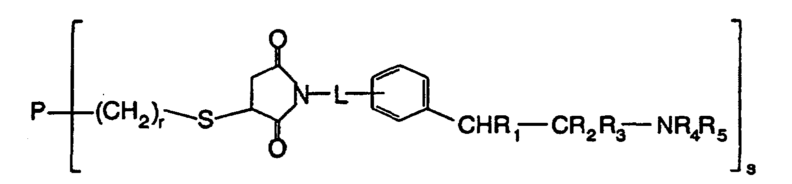 wobei L [-(CH₂)ₒ-NH-C(O)-]ₚ-(CH₂)_{q}- ist, o, p, q und R₁ bis R₅ wie vorstehend definiert sind, P ein Konjugationspartner ist, r 0 bis 10 ist und s 1 bis 40 ist, oder ein Salz davon.

Bevorzugte Ausführungsformen betreffend die genaue Struktur des Linkers sowie die Bedeutung der Reste R₁ bis R₅ entsprechen den vorstehend für das aktivierte Amphetaminderivat genannten.

P kann ein Polypeptid sein wie beispielsweise ein Träger oder ein Detektions-Polypeptid wie etwa ein Immunglobulin oder Enzym. Falls P ein Träger ist, wird durch die Konjugation z.B. ein Immunogen oder ein Polyhapten erhalten.

In manchen Anwendungen für die erfindungsgemäßen Konjugate, wie beispielsweise in Immunassays, kann es bevorzugt sein, daß der Konjugationspartner an eine Festphase gebunden oder mit einer Festphase bindefähig ist. Geeignete Methoden dazu sind dem Fachmann bekannt und werden nicht näher ausgeführt. Bindefähigkeit mit einer Festphase kann mit geeigneten Bindungspaaren, wie beispielsweise Biotin und Avidin/Streptavidin erreicht werden.

Ein weiterer Gegenstand der Erfindung ist ein Verfahren zur Herstellung eines aktivierten Amphetaminderivats, welches dadurch gekennzeichnet ist, daß man in einer ein- oder mehrstufigen Synthese am aromatischen Ring des Amphetamins einen Linker, welcher zur Umsetzung mit Maleinimidoalkylamin geeignet ist, einführt, wobei während der Synthese die Amingruppe der Amphetamin-Ethylamin-Seitenkette geschützt ist, das entstandene Derivat mit Maleinimidoalkylamin umsetzt, die Schutzgruppe entfernt und das aktivierte Amphetaminderivat isoliert.

Der Schutz der Amphetamin-Ethylamin-Seitenkette erfolgt dabei durch übliche Schutzgruppen und es versteht sich, daß die Schutzgruppen lediglich bei Syntheseschritten vorhanden sein müssen, bei denen eine Reaktion der Amingruppe stattfindet bzw. zu erwarten ist. Nach der Isolierung des aktivierten Amphetaminderivats kann die Vollständigkeit der Schutzgruppenabspaltung überprüft werden und das Isolat ggf. weiter aufgereinigt werden um ein Produkt zu erhalten, das im wesentlichen frei von Schutzgruppen ist.

Bevorzugt ist die zur Umsetzung mit Maleinimidoalkylamin geeignete Gruppe ein N-Hydroxysuccinimidester. Die Herstellung von Linkem, die eine N-Hydroxysuccinimidester-Gruppe enthalten, ist aus dem Stand der Technik prinzipell bekannt. Weitere Ausführungsformen werden in den Beispielen dieser Erfindung explizit angeben und spezifische Strukturbeispiele für derartige N-Hydroxysuccinimidester sind den beigefügten Figuren (Verbindung **7** bzw. **16)** zu entnehmen.

Ein nochmal weiterer Gegenstand der vorliegenden Erfindung ist ein Verfahren zur Herstellung eines Konjugats, welches dadurch gekennzeichnet ist, daß man ein aktiviertes Amphetaminderivat wie vorstehend definiert mit einem Reagenz P- [- (CH₂)ᵣ-SH] wobei P, r und s wie vorstehend definiert sind, unter solchen Bedingungen miteinander umsetzt, daß eine Addition der -SH-Gruppen an die Doppelbindung in der Maleinimidfunktionalität stattfindet, und das Reaktionsprodukt isoliert.

Der Vorteil der auf diese Weise hergestellten erfindungsgemäßen Konjugate gegenüber herkömmlich verwendeten Konjugaten besteht darin, daß nach dem Konjugationsschritt eine Abspaltung der Schutzgruppe, die zu Inaktivierung des Konjugatpartners führen kann, nicht erforderlich ist. Weiterhin kann durch die zur Verfügung stehenden analytischen Verfahren die Vollständigkeit der Schutzgruppenentfernung gewährleistet werden. Dadurch wird sichergestellt, daß die Amphetamingruppe für nachfolgende Verwendungen quantitativ zur Verfügung steht.

Weiterhin betrifft die Erfindung die Verwendung eines erfindungsgemäßen Konjugats in einem Verfahren zur Herstellung von gegen Amphetamine gerichteten Antikörpern sowie bei der Bestimmung von Amphetaminen.

Die Herstellung von Antikörpern erfolgt duch Immunisierung eines Versuchstiers mit dem Immunogen, d.h. einem erfindungsgemäßen Konjugat, wobei der Konjugationspartner ein Träger ist.

Die Immunisierung erfolgt auf eine übliche dem Fachmann bekannte Weise, bevorzugt verabreicht man dem Versuchstier das Immunogen in Kombination mit einem Adjuvans. Besonders bevorzugt verwendet man als Adjuvans Freund'sches Adjuvans oder Aluminiumhydroxyd zusammen mit Bordetella pertussis. Die Immunisierung erfolgt vorzugsweise über mehrere Monate mit mindestens vier Immunisierungen in 4- bis 6-wöchigem Abstand. Die Injektion'des Immunogens erfolgt vorzugsweise intraperitoneal.

Aus den derart immunisierten Tieren werden polyklonale Antikörper gewonnen, die nach üblichen Isolierungs- und Aufarbeitungsverfahren gereinigt werden. Alternativ können auch zur Herstellung von monoklonalen Antikörpern B-Lymphozyten gewonnen werden, die mit einer permanenten Myelomzellinie gemäß den bekannten Verfahren von Köhler und Milstein (Nature 256 (1975), 495-497) fusioniert werden. Die hierbei gebildeten Primärkulturen von Hybridzellen werden in üblicherweise z.B. unter Verwendung eines handelsüblichen Zellsorters oder durch "limited dilution" kloniert und die Kulturen weiter verarbeitet, die in einem geeigneten Testverfahren positiv gegen ein Amphetamin reagieren.

Derart erhaltene Antikörper werden vorteilhaft in Immunoassays verwendet. Insbesondere werden derartige Antikörper an eine Markierung oder festphasenseitig, d.h. an eine Festphase gebunden oder damit bindefähig verwendet.

Bevorzugt wird der Immunoassay in Form eines heterogenen Immunoassays durchgeführt, besonders bevorzugt auf einem Chromatographieteststreifen, wie er beispielsweise in DE-OS-44 39 429 oder DE-OS-40 24 919 beschrieben ist.

Ein solcher Teststreifen enthält bevorzugt auf einer Trägerfolie hintereinander angeordnete saugfähige Zonen: eine Analytaufgabezone, eine Konjugatzone auf der ein markierter Bindungspartner und gegebenenfalls ein Bindungspartner mit einer spezifischen Bindestelle (z.B. Biotin) für die nachfolgende Abfangzone untergebracht ist, eine Abfangzone, auf der ein Abfangreagenz für den Analyten oder ein Analytantikörper oder ein spezifisches Abfangreagenz für eine spezifische Bindungsstelle eines Bindungspartners (z.B. Streptavidin) aufgebracht ist und eine Zielzone in der nicht abgefangene Markierung gemessen wird.

Wird der Immunoassay nach einem Sandwichprinzip durchgeführt, bildet sich nach Aufgabe des Analyten auf die Aufgabezone in der Konjugatzone eine Komplex von Analyt mit einem markierten Antikörper, der über einen weiteren Antikörper, der entweder in der Festphasenzone festphasengebunden ist oder dort über ein spezifisches Bindungspaar, z.B. Biotin-Streptavidin gebunden werden kann, dort immobilisiert wird.

Bevorzugt wird der Immunoassay nach einem IEMA Testprinzip (Immuno Enzymetric Assay) durchgeführt. Die Testdurchführung erfolgt vorzugsweise in einer immunologischen Reaktionszelle, die eine Konjugatzone und eine Abfangzone umfaßt. In der Konjugatzone befindet sich ein Nachweisreagenz, z.B. ein markierter Antikörper, vorzugsweise im Überschuß gegenüber dem zu bestimmenden Analyten. Überschüssige markierte Antikörper werden in der Abfangzone durch Analytanaloga, z.B. festphasengebundene Polyhaptene, abgefangen, während markierte Analyt-Antikörper-Komplexe im Durchlauf der Reaktionszelle nachgewiesen werden. Dabei ergibt sich eine steigende Eichkurve, d.h. das Meßsignal nimmt mit der Analytkonzentration zu.

Ein weiterer Gegenstand der Erfindung ist ein Reagenzienkit, das ein erfindungsgemäßes Amphetaminderivat oder/und Konjugat umfaßt. Ein derartiges Reagenzienkit kann im einfachsten Fall lediglich ein aktiviertes Amphetaminderivat umfassen, um einem Anwender zu ermöglichen spezifisch Konjugate für seinen besonderen Verwendungszweck herzustellen. Zusätzlich oder alternativ kann das Kit bereits fertige Konjugate enthalten, die in Immunisierungen oder Bestimmungsverfahren für Amphetamine einsetzbar sind. Weiterhin kann das Kit auch übliche Hilfs-, Puffer- und Zusatzstoffe und ggf. anti-Amphetamin Antikörper enthalten. Ein bevorzugtes Reagenzienkit ist der vorstehend beschriebene Chromatographieteststreifen.

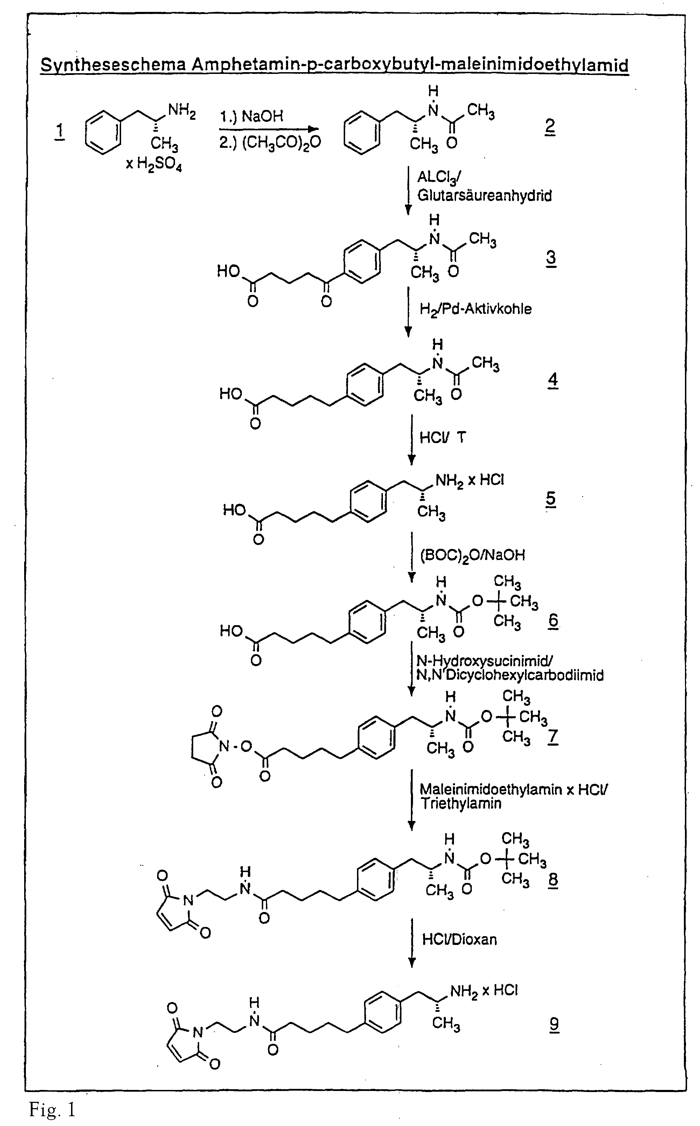

Die Erfindung wird ausführlicher beschrieben durch die nachfolgenden Beispiele in Verbindung mit den beigefügten Figuren, worin
- Figur 1: das Syntheseschema von Amphetamin-p-Carboxybutylmaleinimidoethylamid darstellt und

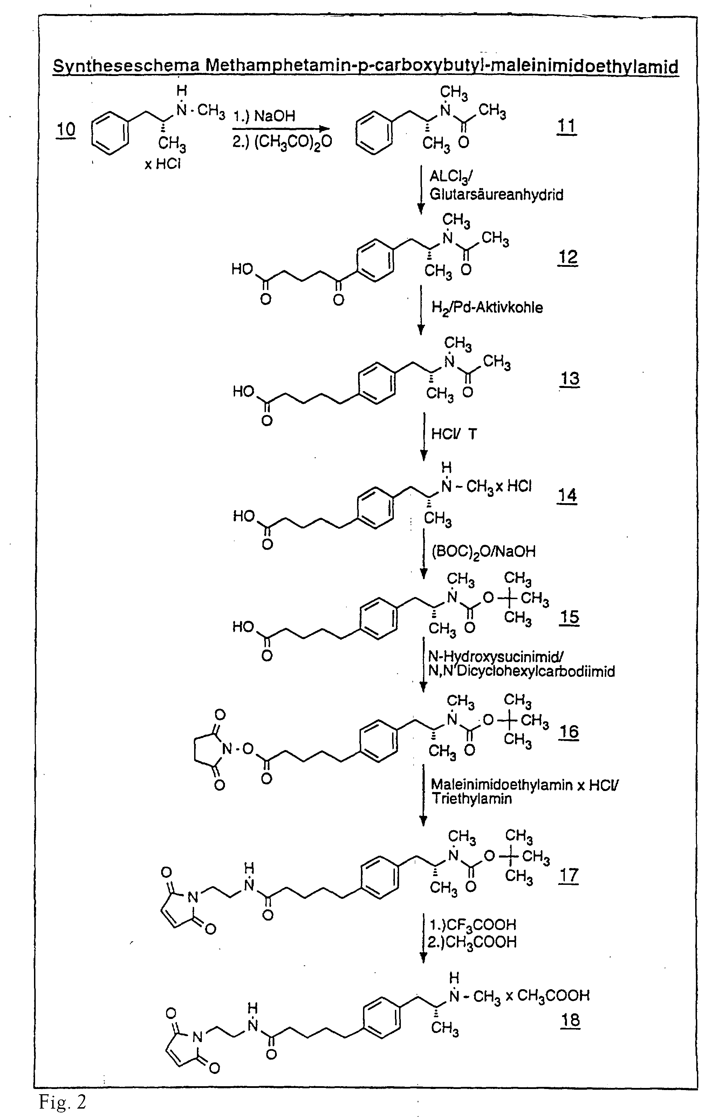

- Figur 2: das Syntheseschema von Methamphetamin-p-Carboxybutyl-maleinimidoethylamid darstellt.

### Beispiele

### Beispiel 1:

### Herstellung von Amphetamin-p-CB-MEA (Amphetamin-p-Carboxybutyl-maleinimidoethylamid) (Hydrochlorid)

### 1. (S)-N-(1-Methyl-2-phenylethyl)acetamid (N-Acetyld-amphetamin) 2

23.3 g (0.1 mol) Dexamphetaminsulfat werden in 75 ml Wasser suspendiert und bei R.T. mit einer Lösung von 10 g (0.25 mol) NaOH in 100 ml Wasser versetzt. Nach kurzem, kräftigem Schütteln scheidet sich ein Öl ab, welches mit 3 x 100 ml Toluol extrahiert wird. Der Extrakt wird mit 50 g K₂CO₃ getrocknet. Anschließend filtriert man über ein Faltenfilter.
Zum Filtrat gibt man 50 ml (0.5 mol) Acetanhydrid und erhitzt die Lösung 1.5 h unter Rückfluß. Danach dampft man am Wasserstrahlvakuum bei 55°C Badtemperatur ein und trocknet den kristallinen Rückstand ca 1 h am Hochvakuum. Das Produkt wird 1-2 h mit 150 ml Diisopropylether digeriert, abgesaugt und 12-14 h im Vakuumtrockenschrank (ca. 150 mbar) bei 40°C getrocknet.
- Ausbeute:: 19.1 g (Theorie: 17.7 g) leicht essigsäurehaltiger, farbloser Feststoff.
- DC:: Kieselgel 60; Essigester/Methanol 1/1 (v/v); R_{f} = 0.86

### 2. N-Acetyl-p-carboxypropylcarbonyl-d-amphetamin 3

Die gesamte unter 1.) erhaltene Menge an Amid **2** (0.1 mol) wird zusammen mit 17.1 g (0.15 mol) Glutarsäureanhydrid in 500 ml frisch über Al₂O₃ destilliertem Methylenchlorid gelöst, die Lösung wird auf 0-5°C gekühlt. Innerhalb von 30 min gibt man unter kräftigem Rühren und fortgesetzter Kühlung des Kolbens auf 0-5°C portionsweise 53.4 g (0.4 mol) AlCl₃ zur Lösung zu. Man rührt 4 h unter Kühlung weiter, dann läßt man die Mischung unter ständigem Weiterrühren auf RT kommen und über Nacht stehen. Der teilweise klumpige, braungelbe Kolbeninhalt wird wieder auf 0-5°C gekühlt und unter Rühren mit 200 ml 3 M HCl versetzt, wobei sich innerhalb 1 h die großen Partikel langsam auflösen und sich ein rotes Öl abscheidet. Die am Kolbenboden stehende Methylenchloridphase wird mit einer Pipette abgesaugt und 250 ml frisches Methylenchlorid zugegeben. Man läßt 15 min weiterrühren, dann wird das organische Lösungsmittel erneut entnommen und mit der ersten Methylenchloridmenge vereinigt. Man extrahiert die organische Phase in einem Scheidetrichter mit 250 ml Wasser. Anschließend wird der wässrige Extrakt mit dem noch im Reaktionskolben befindlichen öligen Rückstand vereinigt. Zu der Mischung gibt man unter Rühren bei RT langsam 1 n NaOH zu, bis die wässrige Phase schwach alkalische Reaktion zeigt (pH 8-9). Man läßt weiterrühren (Zersetzung von gebildetem organischem Säurechlorid), wobei der pH-Wert durch Nachdosieren von NaOH bei etwa 8-9 gehalten wird. Die Mischung bleibt über Nacht bei RT stehen. Anschließend stellt man mit 3 n HCl auf pH 4-5 und gibt 200 ml Dioxan zu, wobei eine fast homogene Lösung entsteht. Diese wird mit 3 x 200 ml Essigester extrahiert. Der Extrakt wird mit ca. 80 g Na₂SO₄ getrocknet und am Rotationsverdampfer eingedampft.
Der ölig braune Rückstand wird in möglichst wenig Essigester/-Methanol 4/1 (v/v) gelöst und auf eine Säule (8.5 x 60 cm, Kieselgel 60, Fa.Merck) aufgetragen. Man eluiert mit Essigester/Methanol 4/1 (v/v) und testet die einzelnen Fraktionen (100 ml) mittels DC auf Kieselgel 60, F₂₅₄ (gleiches Laufmittel) aus. Die das reine Produkt enthaltenden Fraktionen werden vereinigt und das Lösungsmittel am Rotationsverdampfer entfernt. Der Rückstand wird aus 80 ml Wasser/Isopropanol 6/1 (v/v) umkristallisiert, abgesaugt und 12-14 h im Vakuumtrokkenschrank (ca. 150 mbar) bei 40°C getrocknet.
- Ausbeute:: 8.4 g (28.9 % d.Th.) schwach gelbliche Kristalle.
- DC:: Kieselgel 60; Essigester/Methanol 4/1 (v/v); R_{f} = 0.60

### 3. N-Acetyl-p-carboxybutyl-d-amphetamin 4

7.28 g (25 mmol) des Phenylketons **3** werden in 100 ml wasser- und peroxidfreiem THF gelöst und mit 0.5 g frischem Pd/Aktivkohle versetzt. Anschließend wird in einer Glasapparatur (Schüttelente) bei leichtem Überdruck (40-50 mbar) und RT hydriert.
Reaktionsdauer: 1 h
Theoret. H₂-Aufnahme: 1.12 l; Prakt. H₂-Aufnahme: ca. 1.2 1 Anschließend wird die Apparatur mit N₂ belüftet und ausgiebig gespült, um restlichen Wasserstoff auszutreiben. Der Katalysator wird abfiltriert, bleibt dabei aber mit Lösungsmittel benetzt und wird anschließend entsorgt.
Die Lösung wird am Rotationsverdampfer eingedampft und 12-14 h im Vakuumtrockenschrank (ca. 150 mbar) bei 40°C getrocknet. Das Produkt wird ohne weitere Aufreinigung zur nächsten Stufe eingesetzt.
- Ausbeute:: 6.95 g (Theorie: 6.93 g) farbloser Feststoff.
- DC:: Kieselgel 60; Essigester/Eisessig 9/1 (v/v); R_{f} = 0.70

### 4. p-Carboxybutyl-d-amphetamin Hydrochlorid 5

6.93 g (25 mmol) des Acetamids **4** werden in 100 ml conc. HCl 30 h unter Rückfluß gerührt. Danach läßt man langsam auf RT abkühlen und über Nacht stehen. Das ausgefallene Produkt wird abgesaugt, mit 100 ml Aceton digeriert, erneut abgesaugt und 12-14 h im Vakuumtrockenschrank (ca. 150 mbar) bei 40°C getrocknet.
- Ausbeute:: 4.55 g (67 % d.Th.) farbloser Feststoff.
- DC:: Kieselgel 60; Essigester/Eisessig 9/1 (v/v); R_{f} = 0.08

### 5. N-BOC-p-Carboxybutyl-d-amphetamin 6

4.07 g (15 mmol) des Hydrochlorids 5 werden in 120 ml Dioxan/-Wasser 2/1 (v/v) gelöst und mit 22.5 ml 2 n NaOH (45 mmol) versetzt. Man gibt 3.6 g (16.5 mmol) Di(tert.-Butyl)dicarbonat (BOC₂O) in 20 ml Dioxan zu und rührt 2 h bei RT. Eventuell ausfallendes Salz bringt man durch Zugabe von bis zu 30 ml Wasser wieder in Lösung. Der pH-Wert wird überprüft und ggf. mit 2 n NaOH auf 9.0-9.2 nachgestellt. Dann gibt man weitere 0.5 g BOC₂O zu und läßt nochmals 2 h bei gleicher Temperatur weiterrühren. Anschließend stellt man mit 2 m KHSO₄-Lsg. auf pH 2.0, verdünnt mit 200 ml Wasser und extrahiert die Carbonsäure 6 mit 2 x 200 ml Essigester. Den Extrakt wäscht man mit 200 ml Wasser, trocknet mit 30 g Na₂SO₄ und dampft das Lösungsmittel am Rotationsverdampfer ein.
Das verbleibende Öl wird 2 h bei 40°C am Hochvakuum (Rotationsverdampfer) getrocknet.
- Ausbeute:: 5.09 g (Theorie: 5.03 g) zähes, leicht bräunliches Öl.
- DC:: Kieselgel 60; Essigester/Eisessig 9/1 (v/v); R_{f} = 0.86

### 6. N-BOC-d-Amphetamin-p-carboxybutyl-N-hydroxysuccimidester 7

4.02 g (12 mmol) der freien Carbonsäure 6 werden in 60 ml wasserfreiem THF gelöst und mit 1.66 g (14.4 mmol) N-Hydroxysuccinimid und 2.97 g (14.4 mmol) N,N'-Dicyclohexylcarbodiimid versetzt. Man läßt 18 h bei RT rühren, wobei sich klare Kristalle von N,N'-Dicyclohexylharnstoff abscheiden. Diese werden im Anschluß abfiltriert, das Filtrat am Rotationsverdampfer eingedampft und der Rückstand in 50 ml Essigester gelöst, wobei wiederum Harnstoff als Bodensatz verbleibt. Nach neuerlicher Filtration wird die organische Lösung mit 2 x 50 ml Wasser gewaschen, mit ca. 5 g Na₂SO₄ getrocknet und eingedampft. Der Rückstand wird mit Diisopropylether bis zur vollständigen Kristallisation im verschlossenen Kolben digeriert, abgesaugt und 12-14 h im Vakuumtrockenschrank (ca. 150 mbar) bei 40°C getrocknet. Für längere Lagerung empfielt sich zusätzlich Trocknung im Exsikkator über P₂O₅.
- Ausbeute:: 3.31 g (64 % d.Th.) farbloser, feinkristalliner Feststoff.
- DC:: Kieselgel RP-18; Nitromethan/Ethanol 9/1 (v/v); R_{f} = 0.72

### 7. N-BOC-d-Amphetamin-p-carboxybutyl-maleinimidoethylamid 8

3.24 g (7.5 mmol) des Aktivesters 7 werden 50 ml frisch destilliertem DMF gelöst und mit 1.32 g (7.5 mmol) Maleinimidoethylamin Hydrochlorid (MEA x HCl) und 2.25 ml Triethylamin versetzt. Man läßt 1.5 h bei RT rühren, dann werden nochmals 0.27 g MEA x HCl zugegeben. Im Verlauf der Reaktion bildet sich ein Niederschlag von Triethylammoniumhydrochlorid. Nach weiteren 2 h Rühren bei RT wird das Lösungsmittel am Rotationsverdampfer (Ölpumpenvakuum, Badtemperatur: 45°C) abgezogen, der Rückstand mit ca. 20 ml 5% Essigsäure ca. 30 min. digeriert. Der Feststoff wird abgesaugt, in 100 ml Essigester aufgenommen und mit 50 ml Wasser gewaschen. Die organische Phase trocknet man mit ca. 5 g Na₂SO₄ und dampft die Lösung am Rotationsverdampfer (Wasserstrahlvakuum) ein. Man erhält ca 2.5 g Rohprodukt, das durch Chromatographie an einer Kieselsäule (Kieselgel 60, 3.8 x 40 cm; Essigester/Eisessig 99/1 (v/v)) aufgereinigt wird. Die das reine Produkt enthaltenden Fraktionen werden vereinigt, am Rotationsverdampfer eingedampft und der Rückstand ca 1 h mit ca. 30 ml Diisopropylether digeriert. Man saugt ab und trocknet 12-14 h im Vakuumtrockenschrank (ca. 150 mbar) bei 40°C.
- Ausbeute:: 1.76 g (51 % d.Th.) farbloser Feststoff.
- DC:: Kieselgel 60; Essigester/Eisessig 99/1 (v/v); R_{f} = 0.47 Detektion durch Besprühen mit Ninhydrin-Lsg. (Fa.Merck) oder KMnO₄-Lösung (0.1%).

### 8. d-Amphetamin-p-carboxybutyl-maleinimidoethylamid Hydrochlorid (AH,Amphet-p-CB-MEA Hydrochlorid) 9

1.57 g (4 mmol) der BOC-geschützten Maleinimidoverbindung 8 werden in 25 ml 2 m HCl in Dioxan (frisch hergestellt) gelöst und 30 min bei RT ohne Rühren stehengelassen. Das ausgefallene Öl wird 1 h mit 20 ml Essigester digeriert, wobei vollständige Kristallisation eintritt. Man saugt das Produkt ab und trocknet 48 h im Vakuumtrockenschrank (ca. 150 mbar) bei 40°C.
Ausbeute: 1.38 g (88 % d.Th.) farbloser Feststoff.
DC: Kieselgel 60; n-Butanol/Eisessig/Wasser 50/15/25 (v/v/v); R_{f} = 0.56
Detektion durch Besprühen mit Ninhydrin-Lsg. (Fa.Merck) oder KMnO₄-Lösung (0.1%).
HPLC:

| | |
|---|---|
| Säule | Vydac C18/300Å/5µm/4.6x250mm |
| Vorsäule | Nova-Pak C18/Guard-Pak |
| Det.Wellenl. | 225 nm |
| Eluent | A: Millipore-Wasser / 0.1% TFA B: Acetonitril / 0.1% TFA |
| Gradient | 0-80% B in 0-40 min |
| Probenmenge | 20 µl (c = 1 mg/ml) |
| Fluß | 1 ml/min |
| tᵣ | 16.3 min (Verunrein. bei 16.1 u. 17.7 min) |
| Sollgehalt nach HPLC | >90% |

NMR (d₆DMSO) : δ(ppm) = 1.11 (d, J = 6 Hz; 3 H); 1.47 (m; 4 H); 1.99 (m; 2 H); 2.60-3.55 (m; 9 H); 6.97(s; 2 H, -CH=CH-/wichtiges Signal!); 7.14 (s,br; 4 H); 7.92 (tr, s.br; 1 H); Signal -NH₃⁺ variabel (!).

### Beispiel 2:

### Methamphet-p-CB-MEA (Methamphetamin-p-Carboxybutyl-maleinimidoethylamid (Acetat)

### 1. (S)-N-(1-N-Dimethyl-2-phenylethyl)acetamid (N-Acetyld-methamphetamin) 11

18.6 g (0.1 mol) Methamphetamin-Hydrochlorid werden in 75 ml Wasser gelöst und bei R.T. mit einer Lösung von 5 g (0.25 mol) NaOH in 100 ml Wasser versetzt. Nach kurzem, kräftigem Schütteln scheidet sich ein Öl ab, welches mit 3 x 50 ml Toluol extrahiert wird. Der Extrakt wird mit 50 g Na₂SO₄ getrocknet (ca 1-2 min kräftig schütteln), anschließend filtriert man über ein Faltenfilter.
Zum Filtrat gibt man 75 ml (0.5 mol) Acetanhydrid und erhitzt die Lösung 1.5 h unter Rückfluß. Danach dampft man am Wasserstrahlvakuum bei 55°C Badtemperatur ein und löst in 200 ml Essigester. Die organische Phase wird im Scheidetrichter mit 200 ml 1 % NaHCO₃ und anschließend 200 ml Wasser gewaschen. Man zieht das Lösungsmittel am Rotationsverdampfer ab und trocknet das ölige Rohprodukt ca. 1 h bei 40°C am Hochvakuum (Rotationsverdampfer).
- Ausbeute:: 18.1 g (95% d. Theorie) schwach essigsäurehaltiges, leicht bräunliches Öl.
- DC:: Kieselgel 60; Essigester; R_{f} = 0.41

### 2. N-Acetyl-p-carboxypropylcarbonyl-d-methamphetamin 12

Die gesamte unter 1.) erhaltene Menge an Amid 11 (95 mmol) wird zusammen mit 16.26 g (143.5 mmol) Glutarsäureanhydrid in 475 ml frisch über Al₂O₃ destilliertem Methylenchlorid gelöst, die Lösung wird auf 0-5°C gekühlt. Innerhalb von 30 min gibt man unter kräftigem Rühren und fortgesetzter Kühlung des Kolbens auf 0-5°C portionsweise 50.4 g (0.37 mol) AlCl₃ zur Lösung zu. Man rührt 4 h unter Kühlung weiter, dann läßt man die Mischung unter ständigem Weiterrühren auf RT kommen und über Nacht stehen. Der teilweise klumpige, braungelbe Kolbeninhalt wird wieder auf 0-5°C gekühlt und unter Rühren mit 225 ml 3 M HCl versetzt, wobei sich innerhalb 1 h die großen Partikel langsam auflösen und sich ein rotes Öl abscheidet. Die am Kolbenboden stehende Methylenchloridphase wird mit einer Pipette abgesaugt und 280 ml frisches Methylenchlorid zugegeben. Man läßt 15 min weiterrühren, dann wird das organische Lösungsmittel erneut entnommen und mit der ersten Methylenchloridmenge vereinigt. Man extrahiert die organische Phase in einem Scheidetrichter mit 250 ml Wasser. Anschließend wird der wässrige Extrakt mit dem noch im Reaktionskolben befindlichen öligen Rückstand vereinigt und 4 h bei RT gerührt. Anschließend gibt man 200 ml Dioxan zu, wobei eine fast homogene Lösung entsteht. Diese wird mit 3 x 200 ml Essigester extrahiert. Der Extrakt wird mit ca. 80 g Na₂SO₄ getrocknet und am Rotationsverdampfer eingedampft.

Der ölig braune Rückstand wird in möglichst wenig Essigester/-Methanol 4/1 (v/v) + 1% Eisessig gelöst und auf eine Säule (8.5 x 60 cm, Kieselgel 60, Fa.Merck) aufgetragen. Man eluiert mit Essigester/Methanol 4/1 (v/v) + 1% Eisessig und testet die einzelnen Fraktionen (100 ml) mittels DC auf Kieselgel 60, F₂₅₄ (gleiches Laufmittel) aus. Die das reine Produkt enthaltenden Fraktionen werden vereinigt und das Lösungsmittel am Rotationsverdampfer entfernt. Der Rückstand wird in 200 ml Essigester gelöst und zweimal mit je 50 ml Wasser gewaschen. Die organische Lösung trocknet man mit ca. 10 g Na₂SO₄ wasserfrei und entfernt das Lösungsmittel am Rotationsverdampfer.
- Ausbeute:: 6.68 g (23 % d.Th.) gelblicher Feststoff.
- DC:: Kieselgel 60; Essigester/Methanol 4/1 (v/v) + 1% Eisessig; R_{f} = 0.60

### 3. N-Acetyl-p-carboxybutyl-d-methamphetamin 13

6.68 g (21.9 mmol) des Phenylketons **12** werden in 90 ml wasser- und peroxidfreiem THF gelöst und mit 0.5 g frischem Pd/-Aktivkohle versetzt. Anschließend wird in einer Glasapparatur (Schüttelente) bei leichtem Überdruck (40-50 mbar) und RT hydriert. Die Hydrierung bleibt nach etwa 1 h (1/3 des theoretischen H₂-Volumens ist aufgenommen) stehen, worauf nochmals 0.5 g Katalysator zugegeben wird. Es erfolgt starke H₂-Aufnahme und man läßt die Hydrierung bis zum Stillstand weiterlaufen (1-2 h).

Theoret. H₂-Aufnahme: 0.79 l; Prakt. H₂-Aufnahme: ca. 0.81 l

Anschließend wird die Apparatur mit N₂ belüftet und ausgiebig gespült, um restlichen Wasserstoff auszutreiben. Der Katalysator wird abfiltriert, bleibt dabei aber mit Lösungsmittel benetzt und wird anschließend entsorgt.
Die Lösung wird am Rotationsverdampfer eingedampft und das verbleibende Öl anschließend am Hochvakuum (Rotationsverdampfer, Wasserbad 40°C) getrocknet. Das Produkt wird ohne weitere Aufreinigung zur nächsten Stufe eingesetzt.
- Ausbeute:: 7.03 g (Theorie: 6.37 g) farbloses Öl.
- DC:: Kieselgel 60; Essigester/Eisessig 9/1 (v/v); R_{f} = 0.56

### 4. p-Carboxybutyl-d-methamphetamin Hydrochlorid 14

Die gesamte oben erhaltene Menge (7.03 g) bzw. 6.37 g (21.9 mmol) des Acetamids **13** werden in 100 ml conc. HCl 48 h unter Rückfluß gerührt. Danach dampft man am Rotationsverdampfer ein und nimmt den öligen Rückstand in 100 ml Wasser auf. Die Wasserphase wird mit 2 x 30 ml Essigester extrahiert, der Extrakt mit 5 g Na₂SO₄ getrocknet und eingedampft. Er enthält im wesentlichen nicht verseifte Acetylverbindung, die bei Bedarf zu einem weiteren Verseifungsversuch eingesetzt werden kann.

Die oben erhaltene Wasserphase wird mit 1 g Aktivkohle behandelt, danach filtriert, lyophilisiert und das Lyophilisat (ca. 3 g) mit 2 x 100 Diethylether je 30 min digeriert. Das feste Produkt wird abgesaugt und 12-14 h im Vakuumtrockenschrank (ca. 150 mbar) bei 40°C getrocknet.
- Ausbeute:: 2.90 g (46 % d.Th.) leicht gelblich brauner Feststoff.
- DC:: Kieselgel 60; n-Butanol/Eisessig/Wasser 40/10/50 (v/v/v); R_{f} = 0.39 (geringfügige Verunreinigungen bei R_{f} = 0.26 und 0.44 können toleriert werden).

### 5. N-BOC-p-Carboxybutyl-d-methamphetamin 15

2.90 g (10 mmol) des Hydrochlorids **14** werden in 80 ml Dioxan/-Wasser 1/1 (v/v) gelöst und mit 15 ml 2 n NaOH (18 mmol) versetzt. Man gibt 2.65 g (12 mmol) Di(tert.-Butyl)dicarbonat (BOC₂O) in 10 ml Dioxan zu und rührt 2 h bei RT. Anschließend stellt man mit 2 m KHSO₄-Lsg. auf pH 2.0, verdünnt mit 150 ml Wasser und extrahiert die Carbonsäure **6** mit 2 x 100 ml Essigester. Den Extrakt trocknet mit 10 g Na₂SO₄ und dampft das Lösungsmittel am Rotationsverdampfer ein. Das verbleibende Öl wird 2 h bei 40°C am Hochvakuum (Rotationsverdampfer) getrocknet.
- Ausbeute:: 3.40 g (97 % d.Th.) zähes, nahezu farbloses Öl.
- DC:: Kieselgel 60; Essigester/Eisessig 9/1 (v/v); R_{f} = 0.90

### 6. N-BOC-d-Methamphetamin-p-carboxybutyl-N-hydroxysuccimidester 16

3.36 g (10 mmol) der freien Carbonsäure **15** werden in 50 ml wasserfreiem THF gelöst und mit 1.38 g (12 mmol) N-Hydroxysuccinimid und 2.47 g (12 mmol) N,N'-Dicyclohexylcarbodiimid versetzt. Man läßt 18 h bei RT rühren, wobei sich klare Kristalle von N,N'-Dicyclohexylharnstoff abscheiden. Diese werden im Anschluß abfiltriert, das Filtrat am Rotationsverdampfer eingedampft und der Rückstand in 70 ml Essigester gelöst, wobei wiederum Harnstoff als Bodensatz verbleibt. Nach neuerlicher Filtration wird die organische Lösung mit 2 x 30 ml Wasser gewaschen, mit ca. 3 g Na₂SO₄ getrocknet und eingedampft. Der Rückstand wird 1 h mit 50 ml Petolether im verschlossenen Kolben digeriert, das Lösungsmittel dekantiert und das verbleibende Öl mind. 2 h bei 40°C am Hochvakuum (Rotationsverdampfer) getrocknet. Für Lagerung über mehrere Tage wird eine Lagertemperatur von ≤-20°C empfohlen.
- Ausbeute:: 4.02 g (91 % d.Th.) zähes, nahezu farbloses Öl.
- DC:: Kieselgel RP-18; Nitromethan/Ethanol 9/1 (v/v); R_{f} = 0.75

### 7. N-BOC-d-Methamphetamin-p-carboxybutyl-maleinimidoethylamid 17

4.02 g (9 mmol) des Aktivesters **16** werden in 70 ml frisch destilliertem DMF gelöst und mit 1.75 g (9.9 mmol) Maleinimidoethylamin Hydrochlorid (MEA x HCl) und 3.0 ml Triethylamin versetzt. Man läßt 1.5 h bei RT rühren, dann werden nochmals 0.35 g MEA x HCl zugegeben. Im Verlauf der Reaktion bildet sich ein Niederschlag von Triethylammoniumhydrochlorid. Nach weiteren 2 h Rühren bei RT wird das Lösungsmittel am Rotationsverdampfer (Ölpumpenvakuum, Badtemperatur: 45°C) abgezogen, der Rückstand in 50 ml Essigester aufgenommen und die organische Phase mit ca. 50 ml 5% Essigsäure und anschließend mit 50 ml Wasser gewaschen. Die organische Phase trocknet man mit ca. 5 g Na₂SO₄ und dampft die Lösung am Rotationsverdampfer (Wasserstrahlvakuum) ein. Man erhält ca 4.2 g Rohprodukt, das durch Chromatographie an einer Kieselsäule (Kieselgel 60, 5.5 x 47 cm; Essigester/Eisessig 99/1 (v/v)) aufgereinigt wird. Die das reine Produkt enthaltenden Fraktionen werden vereinigt und am Rotationsverdampfer eingedampft. Man trocknet das ölige Produkt ca. 2 h bei 40°C am Hochvakuum (Rotationsverdampfer).
- Ausbeute:: 2.77 g (65 % d.Th.) zähes, nahezu farbloses Öl.
- DC:: Kieselgel 60; Essigester/Eisessig 99/1 (v/v); R_{f} = 0.49 Detektion durch Besprühen mit Ninhydrin-Lsg. (Fa.Merck) oder KMnO₄-Lösung (0.1%) und durch Fluoreszenzlöschung bei λ= 254 nm.

### 8. d-Methamphetamin-p-carboxybutyl-maleinimidoethylamid Acetat 18

2.77 g (5.9 mmol) der BOC-geschützten Maleinimidoverbindung **17** werden in 45 ml 2 m HCl in Dioxan (frisch hergestellt) gelöst und 30 min bei RT ohne Rühren stehengelassen. Man saugt das ausgefallene Produkt (Hydrochlorid) über eine Filternutsche ab und reinigt das Rohprodukt über Säulenchromatographie (Kieselgel 60, 3.8 x 40 cm; Essigester/Eisessig/Wasser 60/30/10 (v/v/v)) auf, wobei Umsalzung zum Acetat eintritt. Die das reine Produkt enthaltenden Fraktionen werden vereinigt und am Rotationsverdampfer bis auf ein Restvolumen von 5-10 ml eingeengt. Man verdünnt mit Wasser auf ein Endvolumen von ca. 100 ml, saugt das ausgefallene Produkt ab und lyophilisiert. Abschließend trocknet man das fertige Produkt 48 h im Vakuumtrockenschrank (ca. 150 mbar) bei 40°C.
Ausbeute: 1.85 g (77 % d.Th.) farbloser Feststoff (ohne Säulenchromatographie).
DC: Kieselgel 60; Essigester/Eisessig/Wasser 60/30/10 (v/v/v) ; R_{f} = 0.39
Detektion durch Besprühen mit Ninhydrin-Lsg. (Fa.Merck) oder KMnO₄-Lösung (0.1%).
HPLC:

| | |
|---|---|
| Säule | Vydac C18/300Å/5µm/4.6x250mm |
| Vorsäule | Nova-Pak C18/Guard-Pak |
| Det.Wellenl. | 225 nm |
| Eluent | A: Millipore-Wasser / 0.1% TFA B: Acetonitril / 0.1% TFA |
| Gradient | 0-80% B in 0-40 min |
| Probenmenge | 20 µl (c = 1 mg/ml) |
| Fluß | 1 ml/min |
| tᵣ | 17.0 min (Verunrein. bei 15.9 u. 18.1 min) |
| Sollgehalt nach HPLC | >90% |

NMR (d₆DMSO) : δ(ppm) = 0.95 (d, J = Hz; 3 H); 1.45 (m; 4H); 1.80 (s; 3H); 1.98 (m; 2 H); 2.36 (s; 3 H); 2.40-3.50 (m; 9 H); 6.96 (s; 2 H, -CH=CH-/wichtiges Signal!); 7.09 (s,br; 4 H); 7.89 (tr, s.br; 1 H); Signal -NH₃⁺ variabel (!).

Bei einem geringfügig höheren Gehalt an Essigsäure kann das Integral des Signals bei δ(ppm) = 1.80 auch bis zu 4.5 H entsprechen (>5 H nicht zulässig, da sonst Essigsäuregehalt zu groß.).

### Beispiel 3:

### Immunisierung und Herstellung von Antiseren

Immunogene werden hergestellt durch Kopplung des aktivierten Amphetaminderivats 9 gemäß Beispiel 1 bzw. des aktivierten Methamphetaminderivats 18 gemäß Beispiel 2 an Keyhole-Limpethemocyanin (KLH).

Diese Kopplung wird wie folgt durchgeführt:

250 mg KLH-Lyophilisat (150 mg Wirkstoff = 5-10⁻⁵ mol) in 20 ml 0,1 M Kaliumphosphatpuffer pH 7,0 lösen, eine Lösung von 24,5 mg (0,1 mmol) N-Succinimidyl-S-acetylthiopropionat (SATP) in 3 ml Dioxan; zutropfen und 3 h bei Raumtemperatur rühren. Anschließend zentrifugieren. Den grauen, leicht opaleszenten Überstand auf eine AcA 202-Säule (ø = 5 cm; 1 = 25 cm; in 0,1 M Kaliumphosphatpuffer pH 7,0) aufgeben und mit 0,1 M Kaliumphosphatpuffer pH 7,0 eluieren (Detektion mit Durchflußphotometer bei 226 nm und Flachbettschreiber).

Ausbeute: 130 mg KLH-(S)ATP (4-10⁻⁵ mmol); η = 3,54 mg/ml; bestimmt mit BCA-Test; BCA Protein Assay Reagent (Pierce; Best.Nr. 23225X)

Die erhaltene Lösung von KLH-(S)ATP mit 1,8 ml 1 M NH₂OH-Lösung versetzen und 1 h bei Raumtemperatur rühren. Anschließend eine Lösung von 12 mg Maleinimidverbindung in 3 ml DMSO zugeben und 16 h bei Raumtemperatur rühren. Den Ansatz zentrifugieren. Den grauen, leicht opaleszenten Überstand auf eine AcA 202-Säule (ø = 5 cm; 1 = 25 cm in 0,1 M Kaliumphosphatpuffer pH 7,0) aufgeben und mit 0,1 M Kaliumphosphatpuffer pH 7,0 eluieren (Detektion mit Durchflußphotometer bei 226 nm und Flachbrettschreiber). Nach der Proteinbestimmung 6 ml Glycerin zugeben.

Ausbeute: 90 mg Immunogen; η = 1,33 mg/ml (bestimmt mit BCA-Test)

Jeweils 5 Schafe werden mit den derart erhaltenen Immunogenen in Freund'schem Adjuvans immunisiert. Die Dosis beträgt 500 µg je Tier. Die Immunisierungen werden über 6 Monate oder länger jeweils in Abständen von 4 Wochen wiederholt.

Monatlich 1x werden von allen Tieren Antiserum-Proben entnommen und auf Anwesenheit von anti-Amphetamin- bzw. anti-Mechamphetamin-Antikörper untersucht. Die Durchführung dieser Messung ist in Beispiel 4 beschrieben. Für weitere Untersuchungen werden solche Antiseren ausgewählt, die in Verdünnung 1:10000 oder höher ausreichend hohes Meßsignal liefern (mindestens 100 mE nach 30 bis 60 Minuten Farbentwicklung). Drei Monate nach Beginn der Immunisierung zeigten die Seren aller Tiere ausreichend hohes Signal.

### Beispiel 4:

### Nachweis von anti-Amphetamin- bzw. anti-Methamphetamin-Antikörpern

### Verwendete Lösungen:

- Beschichtungspuffer:: 50 mM Natriumbicarbonat; 0,09% Natriumazid; pH 9,6
- Inkubationspuffer:: 10 mM Natriumphosphat; 0,1 % Tween 20 (Fa. Brenntag) 0,9% NaCl; 1% Crotein C (Fa. CRODA GmbH); pH 7,4
- Waschlösung:: 0,9% NaCl; 0,1% Tween 20;
- Substratlösung:: Substratlösung Enzymun (Boehringer Mannheim GmbH), enthält 1,9 mM ABTS und 3,2 mM Natriumperborat in Phosphat-Citrat-Puffer pH 4,4) mit 2 mg/ml Vanillin.

### Durchführung:

### Beschichtung:

Mikrotiterplatten (Maxisorp F96, Fa. Nunc) werden mit Streptavidin (Boehringer Mannheim GmbH) beschichtet, das in der Konzentration 5 µg/ml Protein im Beschichtungspuffer gelöst wird. In jedem Napf der Mikrotiterplatten werden 100 µl dieser Lösung pipettiert. Nach einstündiger Inkubation bei Raumtemperatur unter Schütteln wird die Lösung ausgeschüttet und die Platte dreimal mit Waschlösung gewaschen.

### Synthese von biotinyliertem Amphetamin-BSA-Konjugat:

Amphetamin-p-Carboxybutyl-maleinimidoethylamid-Hydrochlorid **9** bzw.Methamphetamin-p-Carboxybutyl-maleinimidoethylamid-Acetat **18** werden zur Herstellung von Polyhaptenen (Abfangmatrix) nach den Verfahren wie vorstehend in Beispiel 3 beschrieben an biotinyliertes BSA gekoppelt.

### Reaktion der Antikörper mit den Haptenen:

Die Antiseren werden mindestens 1 zu 10000 mit Inkubationspuffer verdünnt. Je 50 µl des verdünnten Serums werden zusammen mit den Hapten-BSA-Lösungen in die Näpfe gegeben und gemischt. Nach einstündiger Inkubation bei Raumtemperatur unter Schütteln wird die Lösung ausgeschüttet und die Platte 3x mit Waschlösung gewaschen.

### Reaktion mit dem Nachweiskonjugat:

Zum Nachweis der Antikörper, die über das biotinylierte Hapten-BSA-Konjugat an die Festphase gebunden sind wird ein Konjugat aus Meerrettichperoxidase und Kaninchenantikörpern gegen IgG aus Schafen verwendet. Das Nachweiskonjugat wird mit Inkubationspuffer auf 20 mU/ml Peroxidaseaktivität verdünnt und diese Lösung auf die Näpfe verteilt (100µl/Napf). Inkubation (60 Minuten) und Waschen erfolgt wie vorstehend beschrieben.

### Substratreaktion:

Alle Näpfe werden mit je 100 µl Substratlösung gefüllt und unter Schütteln inkubiert bis die Farbentwicklung in den Negativ-Kontrollen subjektiv ausreichend erscheint. Danach wird die Extinktion aller Näpfe als Differenzmessung bei den Wellenlängen 405/492 nm bestimmt.

### Beispiel 5:

### Nachweis von anti-(Meth)Amphetamin-Antikörpern

Beispiel 4 wird wiederholt, außer daß anstelle des biotinylierten BSA-Hapten-Konjugats ein Festphasen-gebundenes BSA-Hapten-Konjugat verwendet wird.

### Beispiel 6:

### Nachweis von Amphetaminen

Wie vorstehend in Beispiel 4 beschrieben werden Streptavidinbeschichtete Mikrotiterplatten vorbereitet, sowie biotinylierte Konjugate von anti-Amphetamin-Antikörpern gemäß Beispiel 3.

### Nachweiskonjugat:

Zum Nachweis von Haptenen, die über das Antikörper-Biotin-Konjugat an die Festphase gebunden werden, dient in dem verwendeten kompetitiven Verfahren ein Konjugat aus Meerrettichperoxidäse und Amphetamin bzw. Methamphetamin, das durch Umsetzen der aktivierten Konjugate **9** bzw. **18** mit Meerrettichperoxidase gewonnen wird.

### Reaktion der Antikörper mit den Haptenen:

Jeweils von Amphetamin und Methamphetamin wird eine Verdünnungsreihe in Inkubationspuffer hergestellt. Die Reihe enthält insgesamt 10 verschiedene Konzentrationen in Verdünnungsstufen 1 : 3 beginnend mit der Maximalkonzentration (1 µg/ml). Zum Vergleich wird Inkubationspuffer ohne Hapten eingesetzt.

Je 50 µl Amphetaminlösung, Nachweiskonjugat (20 mU/ml Peroxidaseaktivität) und Lösung des biotinylierten Antikörpers werden auf die Näpfe verteilt. Nach einstündiger Inkubation bei Raumtemperatur unter Schütteln wird die Lösung entfernt und die Platte 3x mit Waschlösung gewaschen.

### Substratrektion:

Alle Näpfe werden mit je 100 µl Substratlösung gefüllt und unter Schütteln inkubiert bis die Farbentwicklung in den Proben ohne Hapten subjektiv ausreichend erscheint. Dann wird die Extinktion aller Näpfe als Differenzmessung bei den Wellenlängen 405/492 nm bestimmt.

### Literatur

1. L.T.Cheng , FEBS Letters 36 (1973), 339-42
2. S.Inayama, Chem.Pharm.Bull. 28 (1977), 2779-82
3. P.A.Mason, J.Immunoassay 4 (1988), 83-98
4. S.A.Eremin, Ther.Drug Mon. 10 (1988), 327-32
5. US-PS 3,690,834, 12.09.72
6. US 4,067,774, 10.01.78
7. 7. EP-0 311 383, 06.10.88
8. EP-0 399 184, 03.04.90
9. EP-0 359 063, 01.09.89
10. G.Cavallini, Il Farmaco 11 (1956), 805-10
11. D.Colbert, Clin.Chem. 31 (1985), 1193-95
12. G.Gallacher, Ther.Drug Mon. 11 (1989), 607-11
13. K.Aoki, Forensic Sci.Int. **44** (1990), 245-55
14. K.Terazawa, J.Immunoassay 12 (1991), 277-291
15. EP-0 279 213, 22.01.88
16. US-PS 401,646, 05.04.77
17. 17. US-PS 3,878,187, 15.04.75
18. EP-0 375 422, 21.12.89
19. US-PS 4,041,076 09.08.77
20. US-PS 4,329,281, 11.05.82
21. EP-0 386 644, 02.03.90
22. T.Niwaguchi, J.Forensic Sci. 27 (1982), 592-97
23. T.Usagawa, J.Immunol.Meth. 119 (1989),111-15
24. WO90/15798, 16.06.90

## Patentansprüche

1. Aktiviertes Amphetaminderivat, **gekennzeichnet durch** die Strukturformel 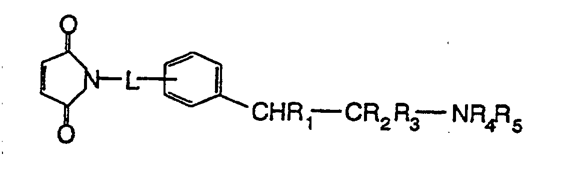 wobei bei R₁, H oder OH ist,
R₂, R₃, R₄ und R₅ unabhängig voneinander H, CH₃, oder C₂H₅ sind und L ein Linker mit einer Kettenlänge von 4-30 Atomen ist, wobei die Linkerkette C-Atome'und gegebenenfalls als Heteroatome N, O und S enthält und wobei die Atome; der Linkerkette der Linkergruppen ausgewählt sind aus -C(R₆)₂-, CR₆OR₆-, -C(O)-, -CR₆=, -C≡, -NR₆-, N=, -O- und -S-, wobei R₆ jeweils unabhängig voneinander H oder C₁ bis C₅ Alkyl darstellt, oder ein Salz davon.

2. Aktiviertes Amphetaminderivat nach Anspruch 1,
**dadurch gekennzeichnet,**
**daß** die Atome in der Linkerkette ausgewählt sind aus-CH₂-, -NH-, -CHOH-, -C(O)-, -CH=, -N=, -C≡, -O- und-S-.

3. Aktiviertes Amphetaminderivat nach einem der vorhergehenden Ansprüche,
**dadurch gekennzeichnet,**
**daß** R₅ H ist.

4. Aktiviertes Amphetaminderivat nach einem der vorhergehenden Ansprüche,
**dadurch gekennzeichnet,**
**daß** der Linker in p-Stellung zu der Ethylamin-Seitenkette steht.

5. Aktiviertes Amphetaminderivat nach einem der vorhergehenden Ansprüche,
**dadurch gekennzeichnet,**
däß L [-(CH₂)ₒ-NH-C(O)-]ₚ-(CH₂)_{q}-X- ist,
wobei o unabhängig bei jedem Vorkommen 2 bis 6 ist,
p 1 oder 2 ist,
q 2 bis 10 ist und
X ausgewählt ist aus O, NH und einer Bindung.

6. Aktiviertes Amphetaminderivat nach Anspruch 5,
**dadurch gekennzeichnet,**
**daß** pl ist und X eine Bindung ist.

7. Aktiviertes Amphetaminderivat nach Anspruch 5 oder 6,
**dadurch gekennzeichnet,**
**daß** o 2 ist und q 4 ist.

8. Aktiviertes Amphetaminderivat nach einem der vorhergehenden Ansprüche,
**dadurch gekennzeichnet,**
**daß** R₁, R₃ und R₅ H sind, R₂ CH₃ ist und R₄ H oder CH₃ ist.

9. Konjugat, umfassen mindestens eine Amphetamingruppe und ein Polypeptid als Konjugationspartner,
**dadurch gekennzeichnet,**
**dass** es durch Umsetzen der Maleimidgruppe eines aktivierten Amphetaminderivats nach einem der vorhergehenden Ansprüche mit einer SH-Gruppe des Konjugationspartners erhältlich ist.

10. Konjugat nach Anspruch 9, **gekennzeichnet durch** die Strukturformel 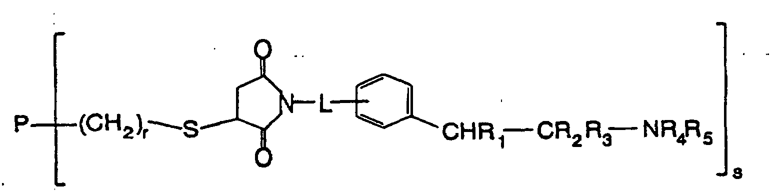 wobei P der Konjugationspartner ist,
R₁ bis R₅ wie in Anspruch 1 definiert sind,
r 0 bis 10 ist, s 1 bis 40 ist und L [- (CH₂)ₒ-NH-C(O) -]ₚ- (CH₂)_{q} ist,
wobei o unabhängig bei jedem Vorkommen 2 bis 6 ist, p 1 oder 2 ist und q 2 bis 10 ist, oder ein Salz davon.

11. Konjugat nach Anspruch 10,
**dadurch gekennzeichnet,**
**daß** p 1 ist.

12. Konjugat nach Anspruch 10 oder 11,
**dadurch gekennzeichnet,**
**daß** o 2 ist und q 4 ist.

13. Konjugat nach einem der Ansprüche 10 bis 12,
**dadurch gekennzeichnet,**
**daß** R₁, R₃ und R₅ H sind, R₂ CH₃ ist und R₄ H oder CH₃ ist.

14. Konjugat nach einem der Ansprüche 10 bis 13,
**dadurch gekennzeichnet,**
**daß** P ausgewählt ist aus Immunglobulinen und Enzymen.

15. Konjugat nach einem der Ansprüche 10 bis 14,
**dadurch gekennzeichnet,**
**daß** es an eine Festphase gebunden oder mit einer Festphase bindefähig ist.

16. Verfahren zur Herstellung eines aktivierten Amphetaminderivats nach einem der Ansprüche 1 bis 8,
**dadurch gekennzeichnet,**
**daß** man in einer ein- oder mehrstufigen Synthese am aromatischen Ring des Amphetamins einen Linker einführt, wobei während der Synthese die Aminogruppe der Amphetamin-Seitenkette geschützt ist, das entstandene Derivat mit Maleinimidolalkylamin umsetzt, die Schutzgruppe entfernt und das aktivierte Amphetaminderivat isoliert.

17. Verfahren zur Herstellung eines Konjugats nach einem der Ansprüche 9 bis 15,
**dadurch gekennzeichnet,**
**daß** man ein aktiviertes Amphetaminderivat nach einem der Ansprüche 1 bis 9 mit einem Reagenz P-[-(CH₂)ᵣ-SH]ₛ, wobei P, r und s wie in Anspruch 11 definiert sind, unter solchen Bedingungen miteinander umsetzt, daß eine Addition der -SH-Gruppen an die Doppelbindung in der Maleinimidfunktionalität stattfindet, und das Reaktionsprodukt isoliert.

18. Verwendeung eines aktivierten Amphetaminderivats nach einem der Ansprüche 1 bis 8 in einem Verfahren zur Herstellung eines Konjugats nach einem der Ansprüche 9 bis 15.

19. Verwendung eines Konjugats nach einem der Ansprüche 10 bis 16 in einem Verfahren zur Herstellung von gegen Amphetamin oder/und Derivate davon gerichteten Antikörpern.

20. Verwendung eines Konjugats nach Anspruch 9 bis 15 in einem Verfahren zur Bestimmung von Amphetamin oder/und von Derivaten davon.

21. Reagenzienkit, umfassend ein aktiviertes Amphetaminderivat nach einem der Ansprüche 1 bis 8 oder/und ein Konjugat nach einem der Ansprüche 9 bis 15.

## Claims

1. Activated amphetamine derivative **characterized by** the structural formula 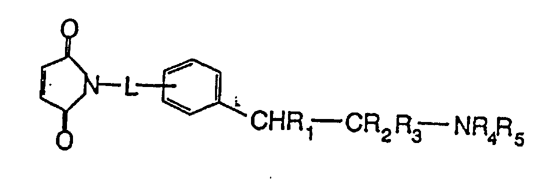 in which R₁ is H or OH,
R₂, R₃, R₄ and R₅ are independently of one another H, CH₃ or C₂H₅ and L is a linker with a chain length of 4-30 atoms, the linker chain containing C atoms and optionally.N, O, and S as heteroatoms and the atoms of the linker chain of the linker groups being selected from -C(R₆)₂-, CR₆OR₆-, -C(O)-, -CR₆=, -C≡, -NR₆-, N=, -O- and -S-, in which R₆ in each case independently of one another represents H or C₁ to C₅ alkyl or a salt thereof.

2. Activated amphetamine derivative as claimed in claim 1,
**wherein**
the atoms in the linker chain are selected from-CH₂-, -NH-, -CHOH-, -C(O)-, -CH=, -N=, -C≡, -O- and -S-.

3. Activated amphetamine derivative as claimed in one of the previous claims,
**wherein**
R₅ is H.

4. Activated amphetamine derivative as claimed in one of the previous claims,
**wherein**
the linker is in the p-position relative to the ethylamine side chain.

5. Activated amphetamine derivative as claimed in one of the previous claims,
**wherein**
L is [-(CH₂)ₒ-NH-C(O)-]ₚ-(CH₂)_{q}-X-, in which o at each occurrence is independently 2 to 6,
p is 1 or 2,
q is 2 to 10 and
X is selected from O, NH and a bond.

6. Activated amphetamine derivative as claimed in claim 5,
**wherein**
p is 1 and X is a bond.

7. Activated amphetamine derivative as claimed in claim 5 or 6,
**wherein**
o is 2 and q is 4.

8. Activated amphetamine derivative as claimed in one of the previous claims,
**wherein**
R₁, R₃ and R₅ are H, R₂ is CH₃ and R₄ is H or CH₃.

9. Conjugate, comprising at least one amphetamine group and a polypeptide as the conjugate partner,
**wherein**
it can be obtained by reacting the maleimide group of an activated amphetamine derivative as claimed in one of the previous claims with an SH group of the conjugation partner.

10. Conjugate as claimed in claim 9, **characterized by** the structural formula 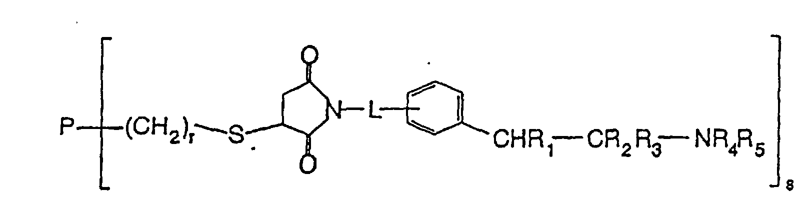 in which P is a conjugation partner,
R₁ to R₅ are defined as in claim 1,
r is 0 to 10, s is 1 to 40 and L is [-(CH₂)ₒ-NH-C(O)-]ₚ-(CH₂)_{q}-, wherein o independently at each occurrence is 2 to 6, p is 1 or 2 and q is 2 to 10 or a salt thereof.

11. Conjugate as claimed in claim 10,
**wherein**
p is 1.

12. Conjugate as claimed in claim 10 or 11,
**wherein**
o is 2 and q is 4.

13. Conjugate as claimed in one of the claims 10 to 12,
**wherein**
R₁, R₃ and R₅ are H, R₂ is CH₃ and R₄ is H or CH₃.

14. Conjugate as claimed in one of the claims 10 to 13,
**wherein**
P is selected from immunoglobulins and enzymes.

15. Conjugate as claimed in one of the claims 10 to 14,
**wherein**
it is bound to a solid phase or is capable of binding to a solid phase.

16. Process for the production of an activated amphetamine derivative as claimed in one of the claims 1 to 8,
**wherein**
a linker is introduced on the aromatic ring of the amphetamine in a one-step or multiple-step synthesis wherein the amino group of the amphetamine side chain is protected during the synthesis, the derivative that is formed is reacted with maleinimido alkylamine, the protective group is removed and the activated amphetamine derivative is isolated.

17. Process for the production of a conjugate as claimed in one of the claims 9 - 15,
**wherein**
an activated amphetamine derivative as claimed in one of the claims 1 to 9 is reacted together with a reagent P-[-(CH₂)ᵣ-SH]ₛ in which P, r and s are defined as claim 11 under such conditions that an addition of the SH groups to the double bond in the maleinimide functionality occurs and the reaction product is isolated.

18. Use of an activated amphetamine derivative as claimed in one of the claims 1 to 8 in a process for the production of a conjugate as claimed in one of the claims 9 to 15.

19. Use of a conjugate as claimed in one of the claims 10 to 16 in a process for the production of antibodies directed against amphetamine or/and derivatives thereof.

20. Use of a conjugate as claimed in claims 9 to 15 in a method for the determination of amphetamine or/and of derivatives thereof.

21. Reagent kit comprising an activated amphetamine derivative as claimed in one of the claims 1 to 8 or/and a conjugate as claimed in one of the claims 9 to 15.

## Revendications

1. Dérivé d'amphétamine activé **caractérisé par** la formule structurale 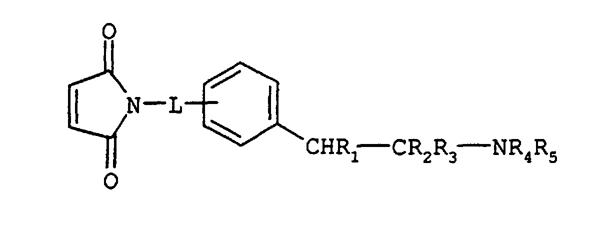 où R₁ est H ou OH,
R₂, R₃, R₄ et R₅ sont indépendamment les uns des autres H, CH₃ ou C₂H₅ et L est un lieur ayant une longueur de chaîne de 4-30 atomes, où la chaîne du lieur contient des atomes de carbone et éventuellement comme hétéroatomes N, O et S et où les atomes de la chaîne du lieur sont choisis parmi -C(R₆)₂-, CR₆OR₆-, -C(O)-, -CR₆=, -C≡, -NR₆-, N=, -O- et -S-, où les R₆ représentent chacun indépendamment des autres H ou alkyle en C₁ à C₅, ou sel de celui-ci.

2. Dérivé d'amphétamine activé selon la revendication 1 **caractérisé en ce que** les atomes dans la chaîne du lieur sont choisis parmi -CH₂-, -NH-, -CHOH-, -C(O)-, -CH=, -N=, -C≡, -O- et -S-.

3. Dérivé d'amphétamine activé selon l'une des revendications précédentes **caractérisé en ce que** R₅ est H.

4. Dérivé d'amphétamine activé selon l'une des revendications précédentes **caractérisé en ce que** le lieur est situé en position p par rapport à la chaîne latérale d'éthylamine.

5. Dérivé d'amphétamine activé selon l'une des revendications précédentes **caractérisé en ce que** L est [-(CH₂)ₒ-NH-C(O)-]ₚ-(CH₂)_{q}-X-,
où o est indépendamment à chaque occurrence 2 à 6,
p est 1 ou 2,
q est 2 à 10 et
X est choisi parmi O, NH et une liaison.

6. Dérivé d'amphétamine activé selon la revendication 5 **caractérisé en ce que** p est 1 et X est une liaison.

7. Dérivé d'amphétamine activé selon la revendication 5 ou 6 **caractérisé en ce que** o est 2 et q est 4.

8. Dérivé d'amphétamine activé selon l'une des revendications précédentes **caractérisé en ce que** R₁, R₃ et R₅ sont H, R₂ est CH₃ et R₄ est H ou CH₃.

9. Conjugué comprenant au moins un groupe amphétamine et un polypeptide comme partenaire de conjugaison, **caractérisé en ce qu'**il peut être obtenu par réaction du groupe maléimide d'un dérivé d'amphétamine activé selon l'une des revendications précédentes avec un groupe SH du partenaire de conjugaison.

10. Conjugué selon la revendication 9
**caractérisé par** la formule structurale 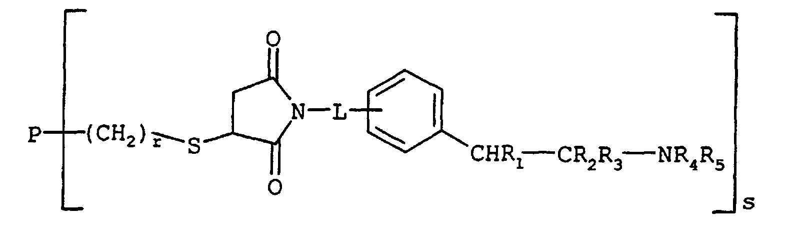 où P est le partenaire de conjugaison,
R₁ à R₅ sont définis comme dans la revendication 1,
r est 0 à 10, s est 1 à 40 et L est [-(CH₂)ₒ-NH-C(O)-]ₚ-(CH₂)_{q}, où o est indépendamment à chaque occurrence 2 à 6, p est 1 ou 2 et q est 2 à 10, ou sel de celui-ci.

11. Conjugué selon la revendication 10 **caractérisé en ce que** p est 1.

12. Conjugué selon la revendication 10 ou 11 **caractérisé en ce que** o est 2 et q est 4.

13. Conjugué selon l'une des revendications 10 à 12 **caractérisé en ce que** R₁, R₃ et R₅ sont H, R₂ est CH₃ et R₄ est H ou CH₃.

14. Conjugué selon l'une des revendications 10 à 13 **caractérisé en ce que** P est choisi parmi les immunoglobulines et les enzymes.

15. Conjugué selon l'une des revendications 10 à 14 **caractérisé en ce qu'**il est lié à une phase solide ou peut être lié à une phase solide.

16. Procédé de production d'un dérivé d'amphétamine activé selon l'une des revendications 1 à 8 **caractérisé en ce que**, dans une synthèse en une ou plusieurs étapes, on introduit un lieur sur le cycle aromatique de l'amphétamine, où, pendant la synthèse, le groupe amino de la chaîne latérale de l'amphétamine est protégé, on fait réagir le dérivé formé avec une maléimidoalkylamine, on retire le groupe protecteur et on isole le dérivé d'amphétamine activé.

17. Procédé de production d'un conjugué selon l'une des revendications 9 à 15 **caractérisé en ce que** l'on fait réagir l'un avec l'autre un dérivé d'amphétamine activé selon l'une des revendications 1 à 9 et un réactif P-[-(CH₂)ᵣ-SH]ₛ où P, r et s sont définis comme dans la revendication 11, dans des conditions telles qu'il se produit une addition des groupes -SH à la double liaison dans la fonctionnalité maléimide, et on isole le produit de la réaction.

18. Utilisation d'un dérivé d'amphétamine activé selon l'une des revendications 1 à 8 dans un procédé de production d'un conjugué selon l'une des revendications 9 à 15.

19. Utilisation d'un conjugué selon l'une des revendications 10 à 16 dans un procédé de production d'anticorps dirigés contre l'amphétamine et/ou des dérivés de celle-ci.

20. Utilisation d'un conjugué selon les revendications 9 à 15 dans un procédé de détermination d'amphétamine et/ou de dérivés de celle-ci.

21. Kit de réactifs comprenant un dérivé d'amphétamine activé selon l'une des revendications 1 à 8 et/ou un conjugué selon l'une des revendications 9 à 15.
